# EUROPÄISCHE PATENTSCHRIFT

(11) **EP 3 428 692 B1**
(45) Veröffentlichungstag und Bekanntmachung des Hinweises auf die Patenterteilung: **10.03.2021**
(21) Anmeldenummer: 17180540.1
(22) Anmeldetag: 10.07.2017
(51) Int. Cl.: G01T 1/24, H01L 27/146

(54) **RÖNTGENDETEKTOR MIT ZWISCHENEINHEIT UND AUSWERTEEBENE**
X-RAY DETECTOR WITH INTERMEDIATE UNIT AND EVALUATION LEVEL
DÉTECTEUR DE RAYONS X COMPRENANT UNE UNITÉ INTERMÉDIAIRE ET NIVEAU D'ÉVALUATION

(43) Veröffentlichungstag der Anmeldung: 16.01.2019
(73) Patentinhaber: Siemens Healthcare GmbH, 91052 Erlangen (DE)
(72) Erfinder: ERGLER, Thorsten, 91054 Erlangen (DE); GEYER, Harald, 91088 Bubenreuth (DE); WREGE, Jan, 91056 Erlangen (DE)

(56) Entgegenhaltungen:
- DE-A1-102007 022 197
- US-B1- 6 510 195

## Beschreibung

Die Erfindung betrifft einen Röntgendetektor, ein medizinisches Gerät und ein Verfahren zur Herstellung des Röntgendetektors, wobei mindestens einem Konverterelement eine Zwischeneinheit und eine Auswerteebene zugeordnet sind.

In der Röntgenbildgebung, beispielsweise in der Computertomographie, der Angiographie oder der Radiographie, können zählende direkt-konvertierende Röntgendetektoren oder integrierende indirekt-konvertierende Röntgendetektoren verwendet werden.

Die Röntgenstrahlung oder die Photonen können in direkt-konvertierenden Röntgendetektoren durch ein geeignetes Konvertermaterial in elektrische Pulse umgewandelt werden. Als Konvertermaterial können beispielsweise CdTe, CZT, CdZnTeSe, CdTeSe, CdMnTe, InP, TlBr2, HgI2, GaAs oder andere verwendet werden. Die elektrischen Pulse werden von einer Auswerteelektronik, beispielsweise einem integrierten Schaltkreis (Application Specific Integrated Circuit, ASIC), bewertet. In zählenden Röntgendetektoren wird einfallende Röntgenstrahlung durch Zählen der elektrischen Pulse, welche durch die Absorption von Röntgenphotonen im Konvertermaterial ausgelöst werden, gemessen. Die Höhe des elektrischen Pulses ist in der Regel proportional zur Energie des absorbierten Röntgenphotons. Dadurch kann eine spektrale Information durch den Vergleich der Höhe des elektrischen Pulses mit einem Schwellwert extrahiert werden. Die Auswerteeinheit kann mit sogenannten Durchkontaktierungen, auch TSV genannt (engl.: through silicon via), versehen sein, so dass diese die in der Auswerteeinheit verarbeiteten, insbesondere digitalen, Signale bzw. Zählwerte an der dem Konverterelement abgewandten Seite zu einem Substrat weiterleiten, in dem eine Umverdrahtung erfolgt und die, insbesondere digitalen, Signale über einen Stecker via Flachbandkabel abgegriffen werden können.

Die Röntgenstrahlung oder die Photonen können in indirektkonvertierenden Röntgendetektoren durch ein geeignetes Konvertermaterial in Licht und mittels Photodioden in elektrische Pulse umgewandelt werden. Als Konvertermaterial werden häufig Szintillatoren, beispielsweise GOS (Gd2O2S), CsJ, YGO oder LuTAG, eingesetzt. Szintillatoren werden insbesondere in der medizinischen Röntgenbildgebung im Energiebereich bis 1MeV eingesetzt. Üblicherweise werden sogenannte indirekt-konvertierende Röntgendetektoren, sogenannte Szintillatordetektoren, verwendet, bei denen die Konvertierung der Röntgen- oder Gammastrahlen in elektrische Signale in zwei Stufen erfolgt. In einer ersten Stufe werden die Röntgen- oder Gammaquanten in einem Szintillatorelement absorbiert und in optisch sichtbares Licht umgewandelt, dieser Effekt wird Lumineszenz genannt. Das durch Lumineszenz angeregte Licht wird anschließend in einer zweiten Stufe durch eine mit dem Szintillatorelement optisch gekoppelten ersten Photodiode in ein elektrisches Signal umgewandelt, über eine Auswerte- oder Ausleseelektronik ausgelesen und anschließend an eine Recheneinheit weitergeleitet.

Aus der Druckschrift DE 10 2014 213 734 A1 ist eine bildgebende Vorrichtung für elektromagnetische Strahlung, insbesondere für Röntgen- und/oder Gamma-Strahlung, bekannt, welche eine Schichtung aus einer Anzahl von Detektionselementen, einer Anzahl von Auslese-Platinen, und einer Basisplatine umfasst, wobei das oder jedes Detektionselement mit jeweils einer Auslese-Platine über eine Mehrzahl von ersten Löt-Kontaktierungen elektrisch kontaktiert ist, wobei die oder jede Auslese-Platine eine Mehrzahl von Durchkontaktierungen aufweist, und wobei die oder jede Auslese-Platine mit der Basisplatine über eine Mehrzahl von zweiten Löt-Kontaktierungen elektrisch kontaktiert ist.

Aus der Druckschrift DE 10 2014 221 829 A1 ist ein Verfahren zur Herstellung eines Sensorboards für ein Detektormodul bekannt, wobei eine Mehrzahl von Ausleseeinheiten bereitgestellt wird, wobei die Ausleseeinheiten in einem Stapelaufbau jeweils auf einer gemeinsamen Sensorschicht positioniert werden, und wobei nach erfolgter Positionierung aller Ausleseeinheiten diese unter Ausbildung eines Hybrids gemeinsam an der Sensorschicht fixiert werden.

Aus der Druckschrift DE 10 2014 225 396 B3 ist ein Sensorboard für ein Detektormodul bekannt, umfassend in einem Stapelaufbau zumindest eine Ausleseeinheit und eine in Stapelrichtung beabstandet zu der Ausleseeinheit angeordnete Sensorschicht, wobei die Sensorschicht in einer Längsrichtung quer zur Stapelrichtung in zumindest einem Randbereich einen Überstand gegenüber der Ausleseeinheit aufweist, wobei der durch die Beabstandung zwischen der Sensorschicht und der Ausleseeinheit vorhandene Zwischenraum derart mit einem ausgehärteten Füllmaterial verfüllt ist, dass zumindest ein Randbereich der Sensorschicht frei von dem Füllmaterial ist.

Die Druckschrift DE 10 2007 022 197 A1 offenbart einen Röntgendetektor aufweisend ein Detektionselement, ein Umkontaktierungssubstrat und zumindest ein elektronisches Bauelement, wobei das Detektionselement und das Bauelement sich gegenüberliegend auf einander abgewandten Seiten des Umkontaktierungssubstrats aufgebracht sind und die Detektionsfläche des Detektionselement größer ist als die Montagefläche des Bauelements.

Aus der Druckschrift US 6 510 195 B1 ist ein Röntgendetektor für ein CT-Gerät bekannt, umfassend eine Mehrzahl an Detektorelementen, welche mit einem Photodiodenarray gekoppelte Szintillatorkristalle aufweisen und wobei das Photodiodenarray auf einem Trägersubstrat aufgebracht ist, welches elektrisch leitende Verbindungen zu Kontakten auf der Rückseite des Auslesesubstrats für die Signalübertragung zur Ausleseelektronik bereitstellt.

Die Erfinder haben erkannt, dass das Konvertermaterial sensitiv auf Spannungen bzw. Spannungsunterschiede reagieren kann, wodurch vor allem im Bereich des Kreuzungspunktes zwischen benachbarten Auswerteeinheiten und entlang der Kanten der benachbarter Auswerteeinheiten ein sogenanntes Driftverhalten auftreten kann, welches sich im elektrischen Puls bzw. im elektrischen Signal des Konverterelements zeigt und somit zur Verfälschung des Signals führt. Das elektrische Signal kann beispielsweise verstärkt oder abgeschwächt sein. Dieses Driftverhalten kann insbesondere aus Spannungen eines ausgehärteten Füllmaterials resultieren.

Es ist Aufgabe der Erfindung, einen Röntgendetektor, ein medizinisches Gerät und ein Verfahren zur Herstellung des Röntgendetektors anzugeben, welche eine Reduktion von mechanischen Spannungen im Stapelaufbau und eine Homogenisierung der elektrischen Signale entlang der flächigen Erstreckung des Konverterelements ermöglichen.

Die Aufgabe wird erfindungsgemäß gelöst durch einen Röntgendetektor nach Anspruch 1, ein medizinisches Gerät nach Anspruch nach Anspruch 7 und ein Verfahren zur Herstellung des Röntgendetektors nach Anspruch 9.

Die Erfindung betrifft einen Röntgendetektor aufweisend ein Konverterelement, eine Zwischeneinheit und eine dem Konverterelement zugeordnete Mehrzahl von Auswerteeinheiten. Die Mehrzahl von Auswerteeinheiten ist in einer Auswerteebene angeordnet. Das Konverterelement, die Zwischeneinheit und die Auswerteebene sind in einer Stapelanordnung angeordnet. Das Konverterelement und die Auswerteeinheiten sind mittels elektrisch leitender Verbindungen elektrisch leitend verbunden.

Der Röntgendetektor kann bevorzugt ein direkt-konvertierenden bzw. quantenzählender Röntgendetektor sein. Der Röntgendetektor weist eine Stapelanordnung auf. Die Stapelrichtung kann insbesondere im Wesentlichen parallel zur betriebsgemäßen Einfallsrichtung der Röntgenstrahlung ausgerichtet sein. Das Konverterelement kann insbesondere flächenhaft sein. Das Konverterelement kann insbesondere eine flächige Erstreckung aufweisen. Die Flächennormale des Konverterelements kann bevorzugt im Wesentlichen parallel zur Einfallsrichtung der Röntgenstrahlung verlaufen. Die Zwischeneinheit kann insbesondere eine flächige Erstreckung aufweisen. Die Flächennormale der Zwischeneinheit kann bevorzugt im Wesentlichen parallel zur Einfallsrichtung der Röntgenstrahlung verlaufen.

Die Auswerteeinheit kann insbesondere eine flächige Erstreckung aufweisen. Die Flächennormale der Auswerteeinheit kann bevorzugt im Wesentlichen parallel zur Einfallsrichtung der Röntgenstrahlung verlaufen. Die Mehrzahl der Auswerteeinheiten ist in einer Auswerteebene angeordnet. Die Flächennormalen der Mehrzahl von Auswerteeinheiten können insbesondere im Wesentlichen parallel zueinander verlaufen. Die Mehrzahl von Auswerteeinheiten kann insbesondere derart nebeneinander angeordnet sein, dass die Mehrzahl von Auswerteeinheiten eine gemeinsame Ebene bzw. eine gemeinsame flächige Erstreckung aufweisen. Die gemeinsame flächige Erstreckung der Mehrzahl von Auswerteeinheiten kann als flächige Erstreckung der Auswerteebene bezeichnet werden. Die Mehrzahl von Auswerteeinheiten kann insbesondere dem Konverterelement zugeordnet sein. Eine ganzzahlige Anzahl von Auswerteeinheiten kann dem Konverterelement zugeordnet sein.

Die Stapelanordnung weist folgende Einheiten bevorzugt in der folgenden Reihenfolge auf: das Konverterelement, die Zwischeneinheit und die Auswerteebene. Das Konverterelement kann dabei bevorzugt der Röntgenstrahlungsquelle am nächsten angeordnet sein, so dass die Röntgenstrahlung insbesondere direkt auf das Konverterelement einfällt. Das Konverterelement ist mittels elektrisch leitender Verbindungen über die Zwischeneinheit mit der Auswerteebene bzw. der Mehrzahl von Auswerteeinheiten verbunden. Sowohl das Konverterelement als auch die Auswerteeinheiten können derart unterteilt sein, dass ein Detektorelement durch einen Teilbereich des Konverterelements und einen Teilbereich einer Auswerteeinheit gebildet werden. Jeder Teilbereich kann insbesondere nur mit einem Teilbereich einer Auswerteeinheit elektrisch leitend verbunden sein. Besonders vorteilhaft können kurze elektrisch leitende Verbindungen zwischen dem Konverterelement und der Auswerteeinheit sein, um eine Signalbeeinflussung durch Leitungskapazitäten so gering wie möglich zu halten. Daher ist eine räumlich nahe Anordnung von Konverterelement und Auswerteeinheiten besonders vorteilhaft.

Eine Mehrzahl von Auswerteeinheiten, bevorzugt als ASIC ausgebildet, sind mit einem Konverterelement, auch Sensor genannt, elektrisch leitend verbunden. Die elektrisch leitende Verbindung kann auch eine mechanische Verbindung bzw. Stabilisierung umfassen. Um eine bessere Ausbeute bei der Herstellung der Auswerteeinheiten zu erhalten sowie um eine Kostenreduktion zu erreichen, können gegenüber dem Konverterelement flächenmäßig kleiner Auswerteeinheiten verwendet werden. Die flächige Erstreckung der Auswerteeinheiten kann insbesondere um einen ganzzahligen Faktor kleiner als die flächige Erstreckung des Konverterelements ausgebildet sein. Beispielsweise kann die flächige Erstreckung der Auswerteeinheit im Wesentlichen einem Viertel der flächigen Erstreckung des Konverterelements entsprechen. Dem Konverterelement können beispielsweise 2 bis 12 Auswerteeinheiten zugeordnet sein. Dem Konverterelement können bevorzugt 4 Auswerteeinheiten zugeordnet sein.

Zwischen dem Konverterelement, der Zwischenschicht und den dazwischen angeordneten elektrisch leitenden Verbindungen sind Zwischenräume bzw. Lücken ausgebildet. Zwischen der Mehrzahl von Auswerteeinheiten, der Zwischenschicht und den dazwischen angeordneten elektrisch leitenden Verbindungen sind Zwischenräume bzw. Lücken ausgebildet. Zwischen benachbarten Auswerteeinheiten kann ein Hohlraum bzw. eine Lücke ausgebildet sein. Zwischen einer etwaigen Trägereinheit, der Mehrzahl von Auswerteeinheiten und dazwischen angeordneten elektrisch leitenden Verbindungen können Zwischenräume bzw. Lücken ausgebildet sein. Die elektrisch leitenden Verbindungen können beispielsweise als Lotverbindungen, insbesondere sogenannte bump bonds oder copper pillars, oder als Leitklebeverbindung ausgebildet sein. Insbesondere zwischen benachbarten Auswerteeinheiten in der Auswerteebene können an den Eckpunkten der Auswerteeinheiten Lücken zwischen mindestens zwei benachbarten Auswerteeinheiten ausgebildet sein. Insbesondere an Kreuzungspunkten mit vier benachbarten Auswerteeinheiten kann eine kreuzförmige Lücke ausgebildet sein. Die Erfinder haben erkannt, dass durch Anordnen einer Zwischeneinheit zwischen dem Konverterelement und der Auswerteebene Spannungen, in Verbindung mit einem Füllmaterial in den Zwischenräumen, reduziert werden können. Durch Anordnung der Zwischeneinheit zwischen dem Konverterelement und der Auswerteebene können, insbesondere mechanische Spannungen, im Stapelaufbau vermieden werden, so dass die Signale entlang der flächigen Erstreckung des Konverterelements gleichartiger sind. Die Zwischeneinheit kann auch als Interposer bezeichnet werden. Die Zwischeneinheit kann Glas, Silizium, starres oder flexibles Leiterplattenmaterial aufweisen. Die Zwischeneinheit kann im Wesentlichen die gleiche flächige Erstreckung wie ein Konverterelement oder eine Mehrzahl von Konverterelementen aufweisen. Eine Einheit aus Auswerteebene, Zwischeneinheit und Konverterelement kann als hybrider Aufbau bzw. Hybrid bezeichnet werden. Vorteilhaft kann durch die Zuordnung einer Zwischeneinheit zu mehreren Auswerteeinheiten eine erhöhte mechanische Stabilität erreicht werden. Besonders vorteilhaft kann die Zwischenschicht Spannungen, beispielsweise durch den Schrumpf eines Füllmaterials in den Zwischenräumen, im Röntgendetektor verringern. Das Konverterelement kann beispielsweise mittels einer Lot- oder Klebeverbindung als zumindest Teil der elektrisch leitenden Verbindung mit der Zwischenschicht verbunden sein. Die elektrisch leitenden Verbindungen führen innerhalb der Zwischeneinheit von der dem Konverterelement zugewandten Seite der Zwischeneinheit zur der der Auswerteebene zugewandten Seite der Zwischeneinheit. Die Auswerteebene kann beispielsweise mittels einer Lot- oder Klebeverbindung als zumindest Teil der elektrisch leitenden Verbindung mit der Zwischenschicht verbunden sein. Die elektrisch leitende Verbindung kann beispielsweise in drei Teilabschnitte aufgeteilt sein: vom Konverterelement zur Zwischeneinheit, durch die Zwischeneinheit hindurch und von der Zwischeneinheit zur Auswerteebene. Vorteilhaft kann ein homogenes Driftverhalten im Konverterelement erreicht werden. Vorteilhaft können Driftveränderungen bzw. Driftinhomogenitäten nahe der Lücken zwischen benachbarten Auswerteeinheiten bzw. den Ecken oder/und Kanten von Auswerteeinheiten verringert werden. Vorteilhaft können die Ladungen bzw. elektrischen Pulse entlang der flächigen Erstreckung homogener bzw. gleichartiger registriert werden.

Gemäß einem Aspekt der Erfindung ist der Zwischeneinheit mehr als ein Konverterelement zugeordnet. Ferner kann eine Zwischeneinheit mehr als einem Konverterelement zugeordnet sein. Dabei sind mehrere Auswerteeinheiten einem Konverterelement zugeordnet. Beispielsweise kann der Stapelaufbau zwei in einer Ebene nebeneinander angeordnete Konverterelemente, eine Zwischeneinheit sowie jeweils vier zugeordnete Auswerteeinheiten pro Konverterelement umfassen. Dieser Stapelaufbau kann Teil eines Sensorboards sein, welches mehrere Konverterelemente zu einer Detektoreinheit verbindet.

Die Integration bzw. Verwendung einer Zwischeneinheit, insbesondere eines sogenannten Interposers, zur Schaffung von homogenen Spannungseigenschaften unterhalb des Konverterelements bzw. zwischen dem Konverterelement und der Mehrzahl von Auswerteeinheiten ermöglichen vorteilhaft eine erhöhte mechanische Stabilisierung des Konverterelements.

Erfindungsgemäß sind die Zwischenräume zwischen dem mindestens einen Konverterelement, der Zwischeneinheit und der Mehrzahl von Auswerteeinheiten mit einem Füllmaterial ausgefüllt.

Um eine ausreichende mechanische Stabilisierung des Hybrids bzw. des Stapelaufbaus des Röntgendetektors zu erreichen ist es vorteilhaft, die sich ergebenden Zwischenräume bzw. Lücken zwischen den elektrisch leitenden Verbindungen, beispielsweise Lot- bzw. Klebeverbindungen, mit einem Füllmaterial bzw. einem sogenannten Underfillmaterial zu füllen. Vorteilhaft können die wirkenden Kräfte unter Rotation des Röntgendetektors in einem Computertomographiesysten von den einzelnen elektrischen Verbindungen reduziert werden und flächig verteilt werden.

Durch die mechanische Stabilisierung mittels Unterfüllung und der Aushärtung des Füllmaterials können je nach Position, beispielsweise unterhalb des Auswerteeinheit bzw. an der Kante der Auswerteeinheit bzw. an einem Kreuzungspunkt zwischen benachbarten Auswerteeinheiten, unterschiedlichen Spannungsbedingungen durch den Schrumpf des Füllmaterials hervorgerufen werden.

Durch Kombination der Unterfüllung mit der Zwischeneinheit kann eine größere Auswahl von Füllmaterialen vorteilhaft genutzt werden. Die Spaltabstände bzw. Lückenabstände bzw. Größe der Zwischenräume zwischen benachbarten Auswerteeinheiten kann vorteilhaft freier gewählt werden. Vorteilhaft kann ein Aushärtungsprofil zum Aushärten bzw. Verfestigen des Füllmaterials größere Toleranzen aufweisen.

Vorteilhaft kann die Spannung bzw. der Spannungsunterschied durch die Kombination von Zwischeneinheit und Unterfüllung verringert werden, so dass das Konvertermaterial, beispielsweise an einem Kreuzungspunkt, nicht mit einer Veränderung des Signals einhergeht und das Auftreten von Bereichen mit Driftveränderungen bzw. Driftinhomogenitäten reduziert bzw. vermieden werden kann.

Vorteilhaft kann durch die Zwischenschicht ein gleichmäßiger bzw. homogener Spalt bzw. Abstand zwischen der Zwischenschicht und dem Konverterelement erreicht werden, so dass die Lücken oder der Kreuzungspunkt zwischen benachbarten Auswerteeinheiten durch die Zwischenschicht vom Konverterelement getrennt sind. Das Konverterelement und die Zwischenschicht folgen im Stapelaufbau aufeinander, dadurch kann der Einfluss der Lücken bzw. des Kreuzungspunkts zwischen benachbarten Auswerteeinheiten reduziert bzw. vermieden werden. Die Auswerteebene ist durch die Zwischenschicht vom Konverterelement getrennt. Insbesondere ist die Unterfüllung erfindungsgemäß zwischen Konverterelement und Zwischeneinheit und die Unterfüllung zwischen Zwischeneinheit und Auswerteeinheiten getrennt ausgebildet, so dass etwaig entstehende Spannungen an den Lücken, Kanten bzw. am Kreuzungspunkt zwischen benachbarten Auswerteeinheiten das Konverterelement bzw. das Konverterelement kaum oder nicht beeinflussen. Vorteilhaft können Driftinhomogenitäten bzw. die Beeinflussung der Signale des Konverterelements vermieden werden.

Vorteilhaft kann durch die mechanische Stabilisierung mittels der Zwischeneinheit eine größere Freiheit in der Auswahl des Füllmaterials bzw. Underfillmaterials und der Prozessführung beim Aushärten bzw. Verfestigen des Füllmaterials erreicht werden.

Die Unterfüllung weist ein Füllmaterial auf. Das Füllmaterial kann eine Epoxid-Verbindung, ein Kunststoffmaterial, einen Verbundwerkstoff oder ein (Prä-)Polymer aufweisen. Das Füllmaterial kann ein Bindematerial aufweisen. Es kann eine Matrix aus Bindematerial und Füllstoff ausgebildet sein. Das Füllmaterial kann insbesondere ein Epoxidharz aufweisen. Zum Zeitpunkt des Füllens der Unterfüllung, beispielsweise zwischen Zwischeneinheit und Konverterelement bzw. Auswerteeinheit, kann das Material der Unterfüllung, beispielsweise aufweisend eine Epoxid-Verbindung, ein Epoxidharz oder ein Präpolymer, flüssig oder fließfähig sein. Vorteilhaft kann die Unterfüllung, beispielsweise unter Temperatureinwirkung, aushärten.

Die Unterfüllung bzw. das Füllmaterial kann eine Wärmeleitfähigkeit von mehr als 0,5W/mK, bevorzugt mehr als 2W/mK, besonders bevorzugt mehr als 6W/mK, aufweisen. Das Füllmaterial bzw. die Unterfüllung kann bevorzugt elektrisch isoliert bzw. nicht-leitend sein. Das Füllmaterial kann einen Füllstoff aufweisen. Der Füllstoff kann einen geringen, insbesondere thermischen, Ausdehnungskoeffizienten aufweisen. Der Füllstoff kann beispielsweise Al2O3, SiO2, BN, AlN, TiN, TiO2, PZT (PbZrTiO3), ZrO2 oder YSZ (sogenanntes Yttria-stabilized zirconia) aufweisen. Der Füllstoff kann vorteilhaft zur mechanischen Stabilität des Stapelaufbaus beitragen. Die Konzentration des Füllstoffes kann derart gewählt werden, dass die Viskosität des Füllmaterials, beispielsweise im fließfähigen Zustand, zwischen 3300mPa·s und 65000mPa·s beträgt. Der Durchmesser oder die Größe der Füllstoffpartikel des Füllstoffs können insbesondere kleiner als der Abstand zwischen der Zwischeneinheit und dem Konverterelement bzw. der Auswerteeinheit, beispielsweise kleiner als 33 Prozent, bevorzugt 20 Prozent und besonders bevorzugt 10 Prozent des Abstands, sein. Der Füllstoff kann zur Anpassung des thermischen Ausdehnungskoeffizienten an die benachbarten Einheiten, beispielsweise das Konverterelement, die Auswerteeinheit, die Zwischeneinheit oder eine etwaige Trägereinheit, vorteilhaft angepasst werden. Die Form der Füllstoffpartikel kann beispielsweise sphärisch, rund, eckig oder flockig sein. Durch den gezielten Einsatz von Füllstoffen mit hohen thermischen Leitfähigkeiten wie beispielsweise Diamant, Nanopartikeln, Graphen oder Kohlenstoffnanoröhrchen kann die thermische Leitfähigkeit der Unterfüllung vorteilhaft erhöht werden. Der thermische Ausdehnungskoeffizient der Unterfüllung, des Füllmaterials oder des Bindematerials kann beispielsweise weniger als 100ppm/K und insbesondere weniger als 50ppm/K betragen und bevorzugt im Bereich von 25 bis 30ppm/K liegen.

Gemäß einem Aspekt der Erfindung umfasst der Stapelaufbau ferner eine Trägereinheit. Die Trägereinheit kann derart ausgebildet sein, dass ihr mindestens ein Stapelaufbau, bevorzugt mehrere Stapelaufbauten, zugeordnet sind. Die etwaige Trägereinheit ist mit der Mehrzahl von Auswerteeinheiten bzw. der Auswerteebene zumindest mechanisch, bevorzugt zusätzlich elektrisch leitend, verbunden. Die etwaige Trägereinheit kann vorteilhaft die mechanische Stabilität des Röntgendetektors erhöhen. Die etwaige Trägereinheit kann vorteilhaft derart ausgebildet sein, dass der Röntgendetektor mehrere nebeneinander benachbarte Stapelaufbauten in einer mechanisch stabilen Anordnung aufweist.

Gemäß einem Aspekt der Erfindung weisen die Zwischeneinheit und das mindestens eine Konverterelement eine im Wesentlichen gleiche flächige Erstreckung auf. Vorteilhaft kann die Zwischeneinheit eine plane und insbesondere unterbrechungsfreie flächige Erstreckung räumlich gegenüber dem Konverterelement bzw. parallel zum Konverterelement aufweisen, so dass beispielsweise der Abstand zwischen dem Konverterelement und der Zwischeneinheit über die flächige Erstreckung des Konverterelements gleich ist. Besonders vorteilhaft können sich durch die unterbrechungsfreie flächige Erstreckung der Zwischeneinheit keine oder verringerte Spannungen ausbilden. Vorteilhaft können Driftinhomogenitäten vermieden oder reduziert werden.

Gemäß einem Aspekt der Erfindung weist die Zwischenschicht eine Durchkontaktierung der elektrisch leitenden Verbindungen von der dem Konverterelement zugewandten Seite der Zwischeneinheit zur der Auswerteebene zugewandten Seite der Zwischeneinheit auf. Die Zwischenschicht kann eine, insbesondere reine bzw. einheitliche, Durchleitung bzw. Durchkontaktierung der elektrisch leitenden Verbindungen, d.h. als 1:1-Umverdrahtung, von der dem Konverterelement zugewandten Seite der Zwischeneinheit zur der Auswerteeinheit zugewandten Seite der Zwischeneinheit aufweisen. Dabei kann die räumliche Anordnung bzw. die Dichte der elektrisch leitenden Verbindung auf der dem Konverterelement zugewandten Seite der Zwischeneinheit und der Auswerteeinheit zugewandten Seite der Zwischeneinheit im Wesentlichen gleich sein.

Gemäß einem Aspekt der Erfindung weist die Zwischenschicht eine Umverdrahtung an der dem Konverterelement zugewandten Seite der Zwischeneinheit oder/und der Auswerteebene zugewandten Seite der Zwischeneinheit auf. Die Zwischenschicht kann eine Umverdrahtung an der dem Konverterelement zugewandten Seite der Zwischeneinheit oder/und der Auswerteeinheit zugewandten Seite der Zwischeneinheit mit Durchleitungen bzw. Durchkontaktierungen durch die Zwischeneinheit aufweisen. Dabei kann die räumliche Anordnung bzw. die Dichte der elektrisch leitenden Verbindung sich auf der dem Konverterelement zugewandten Seite der Zwischeneinheit und der Auswerteeinheit zugewandten Seite der Zwischeneinheit unterscheiden.

Gemäß einem Aspekt der Erfindung ist die Zwischeneinheit mehrlagig ausgestaltet. Die Zwischeneinheit kann mehrlagig ausgestaltet sein, insbesondere im Fall einer Umverdrahtung an der dem Konverterelement zugewandten Seite der Zwischeneinheit oder/und der Auswerteeinheit zugewandten Seite der Zwischeneinheit.
Die Erfindung betrifft ferner ein medizinisches Gerät aufweisend einen erfindungsgemäßen Röntgendetektor. Die Vorteile des erfindungsgemäßen Röntgendetektors können auf das medizinische Gerät übertragen werden. Besonders vorteilhaft kann sich die Homogenisierung der elektrischen Signale des Konverterelements auf eine Homogenisierung der Zählwerte und damit auch auf eine Homogenisierung des aus beispielsweise den Zählwerten erstellten Bildes auswirken. Vorteilhaft können durch die Driftinhomogenitäten bedingte Artefakte im Bild reduziert werden. Vorteilhaft können Korrekturen reduziert oder vermieden werden, um die Driftinhomogenitäten auszugleichen.

Gemäß einem Aspekt der Erfindung ist das medizinische Gerät ein Computertomographiesystem. Vorteilhaft können mit Hilfe der Messdaten, beispielsweise Zählwerte, des Röntgendetektors Schichtbilder, dreidimensionale oder vierdimensionale Volumenbilder rekonstruiert werden.

Die Erfindung betrifft ferner ein Verfahren zur Herstellung eines erfindungsgemäßen Röntgendetektors aufweisend die Schritte des Anordnens und des elektrisch leitenden Verbindens. Im Schritt des Anordnens werden das Konverterelement, die Zwischeneinheit und die dem Konverterelement zugeordneten Mehrzahl von Auswerteeinheiten in einer Stapelanordnung angeordnet, wobei die Mehrzahl von Auswerteeinheiten in einer Auswerteebene angeordnet ist. Im Schritt des elektrisch leitenden Verbindens werden das Konverterelement und die Mehrzahl von Auswerteeinheiten elektrisch leitend verbunden.

Im Schritt des Anordnens können das Konverterelement, die Zwischeneinheit und die Mehrzahl von Auswerteeinheiten und gegebenenfalls die etwaige Trägereinheit zueinander in einem Stapelaufbau angeordnet werden. Im Schritt des elektrisch leitenden Verbindens werden das Konverterelement, die Zwischeneinheit und die Mehrzahl von Auswerteeinheiten, beispielsweise mittels Lotverbindungen oder elektrisch leitender Klebeverbindung, verbunden. Der Schritt des elektrisch leitenden Verbindens kann ferner das elektrisch leitende Verbinden der Mehrzahl von Auswerteeinheiten mit der etwaigen Trägereinheit umfassen. Zwischen dem Konverterelement und der Zwischeneinheit bzw. der Zwischeneinheit und der Auswerteeinheit bzw. der Auswerteeinheit und der etwaigen Trägereinheit sind Zwischenräume ausgebildet, welcher im erfindungsgemäßen Schritt des Füllens gefüllt werden. Es kann ferner an der dem Konverterelement abgewandten Seite der Auswerteeinheit ein Substrat oder eine Trägereinheit angeordnet sein, welches beispielsweise mittels Lotverbindungen oder einer elektrisch leitenden Klebeverbindung mit der Mehrzahl von Auswerteeinheiten verbunden ist. Zwischen der Auswerteeinheit und dem Substrat kann ein Zwischenraum ausgebildet sein, welcher im Schritt des Füllens gefüllt werden kann. Vorteilhaft kann mittels der Zwischeneinheit eine erhöhte mechanische Stabilität erreicht werden.

Gemäß einem Aspekt der Erfindung erfolgen das Anordnen und das elektrisch leitende Verbinden schrittweise. Die Schritte des Anordnens und des elektrisch leitenden Verbindens können mehrmals aufeinander folgen, so dass der Stapelaufbau ebenenweise bzw. schichtweise ausgebildet wird. Schrittweise kann im Rahmen der Erfindung ebenenweise bedeuten.

Gemäß einem Aspekt der Erfindung umfasst der Schritt des elektrisch leitenden Verbindens ein Stufenlötverfahren. Das elektrisch leitende Verbinden kann schrittweise erfolgen, so dass der Stapelaufbau in mehreren Verbindungsschritten ausgebildet wird. Dabei können unterschiedliche Verbindungstechniken, beispielsweise Lotverbindung oder Klebeverbindung, verwendet werden. Die elektrisch leitenden Verbindungen können entlang der Stapelrichtung unterschiedliche Materialzusammensetzungen und Eigenschaften, beispielsweise eine unterschiedliche Schmelztemperatur, aufweisen. Es kann ein sogenanntes Stufenlöten zum elektrisch leitenden Verbinden genutzt werden, wobei die Ebenen des Stapelaufbaus nacheinander elektrisch leitend mittels Lotverbindungen mit unterschiedlicher Schmelztemperatur verbunden werden. Bevorzugt kann die niedrigste Schmelztemperatur zwischen dem Konverterelement und der Zwischeneinheit verwendet werden. Beispielsweise kann ein sogenannter Reflow-Lötprozess verwendet werden.

Erfindungsgemäß weist das Verfahren ferner den Schritt des Füllens der Zwischenräume auf. Im Schritt des Füllens werden die Zwischenräumen zwischen dem Konverterelement, dem Zwischenelement und der Mehrzahl von Auswerteeinheiten, und gegebenenfalls zwischen der Mehrzahl von Auswerteeinheiten und der etwaigen Trägereinheit, mit einem Füllmaterial gefüllt. Im Schritt des Füllens kann das Füllmaterial in einem fließfähigen Zustand in den Zwischenraum bzw. die Zwischenräume eingefüllt werden. Das Füllen kann gleichzeitig für alle Zwischenräume einer Ebene bzw. Schicht der Unterfüllung oder schrittweise erfolgen. Vorteilhaft können durch die Unterfüllung bedingte Spannungen reduziert oder vermieden werden.

Gemäß einem Aspekt der Erfindung erfolgt das Füllen schrittweise bzw. ebenenweise. Das Füllen kann zwischen den verschiedenen Ebenen des Stapelaufbaus, also zwischen dem Konverterelement, dem Zwischenelement und der Mehrzahl von Auswerteeinheiten, und gegebenenfalls zwischen der Mehrzahl von Auswerteeinheiten und der etwaigen Trägereinheit, gleichzeitig oder schrittweise, insbesondere nacheinander, eingefüllt werden. Das Füllmaterial ist für die Zwischenräume unterschiedlicher Ebenen unterschiedlich. Beispielsweise kann in den Zwischenräumen zwischen dem Konverterelement und der Zwischeneinheit, dem Zwischenelement und der Mehrzahl von Auswerteeinheiten, und gegebenenfalls zwischen der Mehrzahl von Auswerteeinheiten und der etwaigen Trägereinheit, ein unterschiedliches Füllmaterial verwendet werden. Die unterschiedlichen Füllmaterialien können sich beispielsweise durch Viskosität im fließfähigen Zustand oder/und im verfestigten Zustand, Wärmeleitfähigkeit, thermischem Ausdehnungskoeffizient, Transparenz für Licht im sichtbaren, ultravioletten oder infraroten Bereich oder ähnlichen unterscheiden.

Gemäß einem Aspekt der Erfindung weist das Verfahren ferner den Schritt des Verfestigens des Füllmaterials auf. Das Verfestigen kann gleichzeitig oder schrittweise bzw. ebenenweise erfolgen. Im Schritt des Verfestigens kann das Füllmaterial beispielsweise mittel thermischer Einwirkung oder unter Einwirkung von UV-Strahlung ausgehärtet werden. Durch das Härten wird die Unterfüllung erzeugt. Die Unterfüllung weist im Endzustand ein verfestigtes Füllmaterial auf. Vorteilhaft weist der Röntgendetektor eine erhöhte mechanische Stabilität auf.

Nachfolgend werden Ausführungsbeispiele der Erfindung anhand von Zeichnungen näher erläutert. Hierbei zeigt:
FIG 1 schematisch ein Konzept eines erfindungsgemäßen Röntgendetektors gemäß einer ersten Ausführungsform;
FIG 2 schematisch ein Konzept eines erfindungsgemäßen Röntgendetektors gemäß einer zweiten Ausführungsform;
FIG 3 schematisch eine Draufsicht auf die Zwischeneinheit und die Mehrzahl von Auswerteeinheiten des erfindungsgemäßen Röntgendetektors gemäß der ersten Ausführungsform oder der zweiten Ausführungsform;
FIG 4 schematisch ein Konzept eines erfindungsgemäßen Röntgendetektors gemäß einer dritten Ausführungsform;
FIG 5 schematisch ein Konzept eines erfindungsgemäßen Röntgendetektors gemäß einer vierten Ausführungsform;
FIG 6 schematisch eine Draufsicht auf die Zwischeneinheit und die Mehrzahl von Auswerteeinheiten des erfindungsgemäßen Röntgendetektors gemäß der dritten Ausführungsform oder der vierten Ausführungsform;
FIG 7 schematisch eine Seitenansicht eines erfindungsgemäßen Röntgendetektors gemäß einer fünften Ausführungsform;
FIG 8 schematisch eine Seitenansicht eines erfindungsgemäßen Röntgendetektors gemäß einer sechsten Ausführungsform;
FIG 9 schematisch eine Seitenansicht eines erfindungsgemäßen Röntgendetektors gemäß einer siebten Ausführungsform;
FIG 10 schematisch eine Seitenansicht eines erfindungsgemäßen Röntgendetektors gemäß einer achten Ausführungsform;
FIG 11 schematisch ein Konzept eines erfindungsgemäßen Computertomographen; und
FIG 12 schematisch ein Konzept eines erfindungsgemäßen Verfahrens zur Herstellung eines erfindungsgemäßen Röntgendetektors.

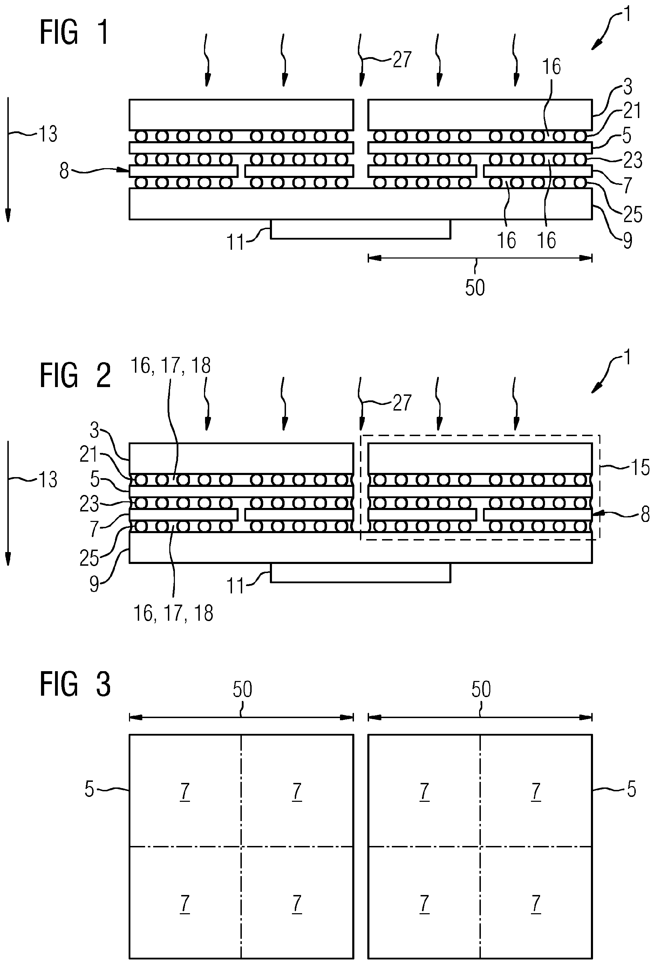

Die Fig. 1 zeigt eine beispielhafte Ausführung des erfindungsgemäßen Röntgendetektors 1 gemäß einer ersten Ausführungsform. Der Röntgendetektor 1 weist ein Konverterelement 3, eine Zwischeneinheit 5 und eine dem Konverterelement 3 zugeordnete Mehrzahl von Auswerteeinheiten 7 auf. Die Mehrzahl von Auswerteeinheiten 7 ist in einer Auswerteebene 8 angeordnet. Das Konverterelement 3, die Zwischeneinheit 5 und die Auswerteebene 8 sind in einer Stapelanordnung angeordnet. Das Konverterelement 3 und die Mehrzahl von Auswerteeinheiten 7 sind mittels elektrisch leitender Verbindungen 21, 23, 25 elektrisch leitend verbunden. Die Zwischeneinheit 5 und das mindestens eine Konverterelement 3 weisen eine im Wesentlichen gleiche flächige Erstreckung 50 auf. Der Stapelaufbau umfasst eine Trägereinheit 9. Die Trägereinheit 9 ist elektrisch leitend mit der Mehrzahl der Auswerteeinheiten 7 verbunden. Die Trägereinheit 9 weist auf der der Auswerteebene 8 abgewandten Seite bzw. Fläche eine Verbindungseinheit 11, beispielsweise einen Stecker, auf. Mittels der Verbindungseinheit 11 werden die Signale des Röntgendetektors 1 ausgelesen und zur Recheneinheit des medizinischen Geräts weitergeleitet.

Der Röntgendetektor 1 ist ein direkt-konvertierenden bzw. quantenzählender Röntgendetektor 1. Der Röntgendetektor 1 weist eine Stapelanordnung mit einer Stapelrichtung 13 auf. Die Stapelrichtung 13 ist im Wesentlichen parallel zur betriebsgemäßen Einfallsrichtung der Röntgenstrahlung 27 ausgerichtet. Das Konverterelement 3 ist flächenhaft. Das Konverterelement 3 weist eine flächige Erstreckung auf. Die Flächennormale des Konverterelements 3 verläuft im Wesentlichen parallel zur Einfallsrichtung der Röntgenstrahlung 27. Die Zwischeneinheit 5 weist eine flächige Erstreckung 50 auf. Die Flächennormale der Zwischeneinheit 5 verläuft im Wesentlichen parallel zur Einfallsrichtung der Röntgenstrahlung 27.

Jede Auswerteeinheit 7 weist eine flächige Erstreckung auf, welche kleiner als die flächige Erstreckung 50 des Konverterelements 3 ist. Die Flächennormale der Mehrzahl von Auswerteeinheiten 7 verläuft im Wesentlichen parallel zur Einfallsrichtung der Röntgenstrahlung 27. Die Mehrzahl der Auswerteeinheiten 7 ist in einer Auswerteebene 8 angeordnet. Die Flächennormalen der Mehrzahl von Auswerteeinheiten 7 verlaufen im Wesentlichen parallel zueinander. Die Mehrzahl von Auswerteeinheiten 7 ist derart nebeneinander angeordnet, dass die Mehrzahl von Auswerteeinheiten 7 eine gemeinsame Ebene bzw. eine gemeinsame flächige Erstreckung 50 aufweisen. Die gemeinsame flächige Erstreckung 50 der Mehrzahl von Auswerteeinheiten 7 wird als flächige Erstreckung 50 der Auswerteebene 8 bezeichnet. Die Mehrzahl von Auswerteeinheiten 7 ist dem Konverterelement 3 zugeordnet. Eine ganzzahlige Anzahl von Auswerteeinheiten 7 ist dem Konverterelement 3 zugeordnet.

Die Stapelanordnung weist folgende Einheiten bevorzugt in der folgenden Reihenfolge auf: das Konverterelement 3, die Zwischeneinheit 5 und die Auswerteebene 8. Das Konverterelement 3 ist der Röntgenstrahlungsquelle am nächsten angeordnet, so dass die Röntgenstrahlung 27 auf das Konverterelement 3 einfällt. Das Konverterelement 3 ist mittels elektrisch leitender Verbindungen 21, 23 über die Zwischeneinheit 5 mit der Auswerteebene 8 bzw. der Mehrzahl von Auswerteeinheiten 7 verbunden. Sowohl das Konverterelement 3 als auch die Auswerteeinheiten 7 sind derart unterteilt, dass ein Detektorelement durch einen Teilbereich des Konverterelements 3 und einen Teilbereich einer Auswerteeinheit 7 gebildet werden. Jeder Teilbereich ist nur mit einem Teilbereich einer Auswerteeinheit 7 elektrisch leitend verbunden.

Eine Mehrzahl von Auswerteeinheiten 7, bevorzugt als ASIC ausgebildet, sind mit einem Konverterelement 3, auch Sensor genannt, elektrisch leitend verbunden. Die flächige Erstreckung der Auswerteeinheiten 7 ist insbesondere um einen ganzzahligen Faktor kleiner als die flächige Erstreckung 50 des Konverterelements 3 ausgebildet. Beispielsweise kann die flächige Erstreckung der Auswerteeinheit 7 im Wesentlichen einem Viertel der flächigen Erstreckung 50 des Konverterelements 3 entsprechen. Dem Konverterelement 3 können beispielsweise 2 bis 12 Auswerteeinheiten 7 zugeordnet sein. Dem Konverterelement 3 sind beispielsweise 4 Auswerteeinheiten zugeordnet.

Zwischen dem Konverterelement 3, der Zwischenschicht 5 und den dazwischen angeordneten elektrisch leitenden Verbindungen 21 sind Zwischenräume 16 bzw. Lücken ausgebildet. Zwischen der Mehrzahl von Auswerteeinheiten 7, der Zwischenschicht 4 und den dazwischen angeordneten elektrisch leitenden Verbindungen 23 können Zwischenräume 16 bzw. Lücken ausgebildet sein. Zwischen benachbarten Auswerteeinheiten 7 ist ein Hohlraum bzw. eine Lücke bzw. ein Zwischenraum 16 ausgebildet. Zwischen einer etwaigen Trägereinheit 9, der Mehrzahl von Auswerteeinheiten 7 und dazwischen angeordneten elektrisch leitenden Verbindungen 25 können Zwischenräume 16 bzw. Lücken ausgebildet sein. Die elektrisch leitenden Verbindungen 21, 23, 25 können beispielsweise als Lotverbindungen, insbesondere sogenannte bump bonds oder copper pillars, oder als Leitklebeverbindung ausgebildet sein. Insbesondere zwischen benachbarten Auswerteeinheiten 7 in der Auswerteebene 8 sind an den Eckpunkten der Auswerteeinheiten 7 Zwischenräume 16 zwischen mindestens zwei benachbarten Auswerteeinheiten 7 ausgebildet. Insbesondere an Kreuzungspunkten mit vier benachbarten Auswerteeinheiten 7 ist ein kreuzförmiger Zwischenraum 16 ausgebildet.

Die Zwischeneinheit 5 kann auch als Interposer bezeichnet werden. Die Zwischeneinheit 5 kann Glas, Silizium, starres oder flexibles Leiterplattenmaterial aufweisen. Die Zwischeneinheit 5 kann im Wesentlichen die gleiche flächige Erstreckung 50 wie ein Konverterelement 3 oder eine Mehrzahl von Konverterelementen 3 aufweisen. Eine Einheit aus Auswerteebene 8, Zwischeneinheit 5 und Konverterelement 3 wird als hybrider Aufbau bzw. Hybrideinheit 15 bezeichnet. Das Konverterelement 3 ist beispielsweise mittels einer Lot- oder Klebeverbindung als zumindest Teil der elektrisch leitenden Verbindung 21 mit der Zwischenschicht 5 verbunden. Die elektrisch leitenden Verbindungen 21, 23 führen innerhalb der Zwischeneinheit 5 von der dem Konverterelement 3 zugewandten Seite der Zwischeneinheit 5 zur der der Auswerteebene 8 zugewandten Seite der Zwischeneinheit 5. Die Auswerteebene 8 kann beispielsweise mittels einer Lot- oder Klebeverbindung als zumindest Teil der elektrisch leitenden Verbindung 23 mit der Zwischenschicht 5 verbunden sein. Die elektrisch leitende Verbindung 21, 23 kann beispielsweise in drei Teilabschnitte aufgeteilt sein: vom Konverterelement 3 zur Zwischeneinheit 5, durch die Zwischeneinheit 5 hindurch und von der Zwischeneinheit 5 zur Auswerteebene 8.

Die Fig. 2 zeigt eine Ausführung des erfindungsgemäßen Röntgendetektors 1. Die Zwischenräume 16 zwischen dem mindestens einen Konverterelement 3, der Zwischeneinheit 5 und der Mehrzahl von Auswerteeinheiten 7 sind mit einem Füllmaterial 18 ausgefüllt. Das Konverterelement 3, die Zwischeneinheit 5 und die Auswerteebene 8 bilden eine Hybrideinheit 15, beispielsweise eine 1:4-Hybrideinheit mit einem Konverterelement und vier Auswerteeinheiten. Die Auswerteebene 8 ist durch die Zwischenschicht 5 vom Konverterelement 3 getrennt. Die Unterfüllung 17 zwischen Konverterelement 3 und Zwischeneinheit 5 und die Unterfüllung 17 zwischen Zwischeneinheit 5 und Auswerteeinheiten 7 sind getrennt ausgebildet.

Die Unterfüllung 17 weist ein Füllmaterial 18 auf. Das Füllmaterial 18 weist eine Epoxid-Verbindung, ein Kunststoffmaterial, einen Verbundwerkstoff oder ein (Prä-)Polymer auf. Das Füllmaterial 18 kann ein Bindematerial aufweisen. Es kann eine Matrix aus Bindematerial und Füllstoff ausgebildet sein. Das Füllmaterial 18 weist insbesondere ein Epoxidharz auf. Zum Zeitpunkt des Füllens der Unterfüllung 17, beispielsweise zwischen Zwischeneinheit 5 und Konverterelement 3 bzw. Auswerteeinheit 7, kann das Material der Unterfüllung 17, beispielsweise aufweisend eine Epoxid-Verbindung, ein Epoxidharz oder ein Präpolymer, flüssig oder fließfähig sein.

Das Füllmaterial 18 kann einen Füllstoff aufweisen. Der Füllstoff kann einen geringen, insbesondere thermischen, Ausdehnungskoeffizienten aufweisen. Der Füllstoff kann beispielsweise Al2O3, SiO2, BN, AlN, TiN, TiO2, PZT (PbZrTiO3), ZrO2 oder YSZ (sogenanntes Yttria-stabilized zirconia) aufweisen. Der Füllstoff kann vorteilhaft zur mechanischen Stabilität des Stapelaufbaus beitragen. Die Konzentration des Füllstoffes kann derart gewählt werden, dass die Viskosität des Füllmaterials 18, beispielsweise im fließfähigen Zustand, zwischen 3300mPa·s und 65000mPa·s beträgt. Der Durchmesser oder die Größe der Füllstoffpartikel des Füllstoffs können insbesondere kleiner als der Abstand zwischen der Zwischeneinheit 5 und dem Konverterelement 3 bzw. der Auswerteeinheit 7, beispielsweise kleiner als 33 Prozent, bevorzugt 20 Prozent und besonders bevorzugt 10 Prozent des Abstands, sein. Der Füllstoff kann zur Anpassung des thermischen Ausdehnungskoeffizienten an die benachbarten Einheiten, beispielsweise das Konverterelement 3, die Auswerteeinheit 7, die Zwischeneinheit 5 oder eine etwaige Trägereinheit 9, angepasst sein. Die Form der Füllstoffpartikel kann beispielsweise sphärisch, rund, eckig oder flockig sein.

Die Fig. 3 zeigt eine beispielhafte Ausführung des erfindungsgemäßen Röntgendetektors 1 gemäß der ersten Ausführungsform oder der zweiten Ausführungsform in einer Draufsicht auf die Zwischeneinheit 5 und die Mehrzahl von Auswerteeinheiten 7. Die flächige Erstreckung 50 der Zwischeneinheit 5 entspricht im Wesentlichen der flächigen Erstreckung 50 der Auswerteebene. Es sind jeweils 2x2 Auswerteeinheiten 7 einer Zwischeneinheit 5 zugeordnet. Die 2x2 Auswerteeinheiten 7 bilden die Auswerteebene.

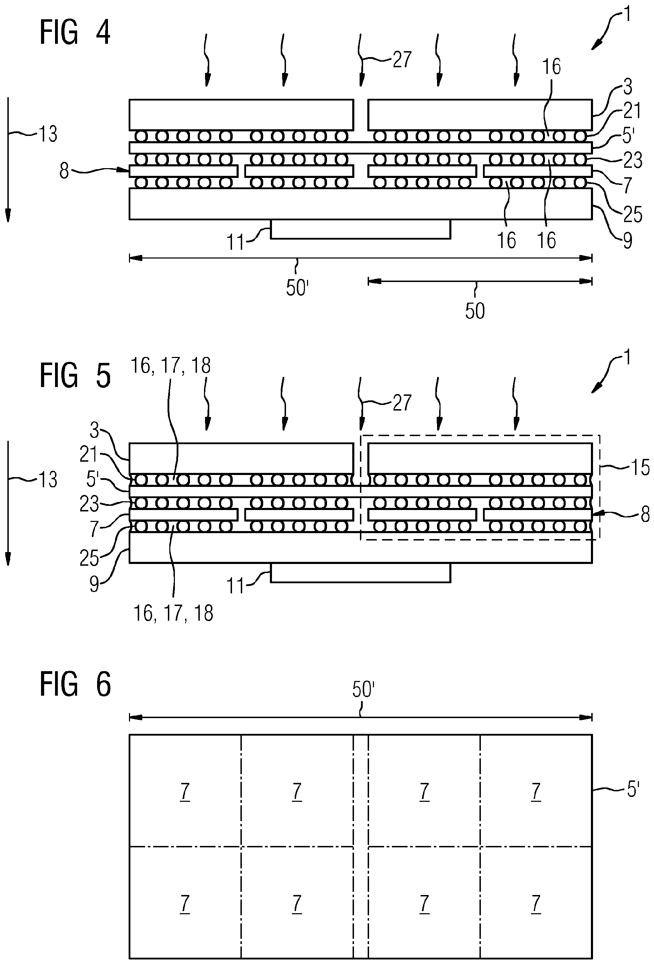

Die Fig. 4 zeigt eine beispielhafte Ausführung des erfindungsgemäßen Röntgendetektors 1 gemäß einer dritten Ausführungsform. Der Röntgendetektor 1 weist ein Konverterelement 3, eine Zwischeneinheit 5' und eine dem Konverterelement 3 zugeordnete Mehrzahl von Auswerteeinheiten 7 auf. Das Konverterelement 3, die Zwischeneinheit 5' und die Auswerteebene 8 sind in einer Stapelanordnung angeordnet. Die Zwischeneinheit 5' und das mindestens eine Konverterelement 3 weisen eine im Wesentlichen gleiche flächige Erstreckung 50' auf. Die Zwischenschicht 5' ist beispielhaft zwei Konverterelementen 3 zugeordnet. Dabei sind beispielshaft vier Auswerteeinheiten 7 einem Konverterelement 3 zugeordnet. Beispielsweise kann der Stapelaufbau zwei in einer Ebene nebeneinander angeordnete Konverterelemente 3, eine Zwischeneinheit 5' sowie jeweils vier zugeordnete Auswerteeinheiten 7 pro Konverterelement 3 umfassen.

Die Fig. 5 zeigt eine beispielhafte Ausführung des erfindungsgemäßen Röntgendetektors 1 gemäß einer vierten Ausführungsform. Die Zwischenräume 16 zwischen dem mindestens einen Konverterelement 3, der Zwischeneinheit 5' und der Mehrzahl von Auswerteeinheiten 7 sind mit einem Füllmaterial 18 ausgefüllt.

Die Fig. 6 zeigt eine beispielhafte Ausführung des erfindungsgemäßen Röntgendetektors 1 gemäß der dritten Ausführungsform oder der vierten Ausführungsform in einer Draufsicht auf die Zwischeneinheit 5' und die Mehrzahl von Auswerteeinheiten 7. Die flächige Erstreckung 50' der Zwischeneinheit 5' entspricht im Wesentlichen der flächigen Erstreckung 50' der Auswerteebene. Es sind 2x4 Auswerteeinheiten 7 der Zwischeneinheit 5` zugeordnet. Die 2x4 Auswerteeinheiten 7 bilden die Auswerteebene.

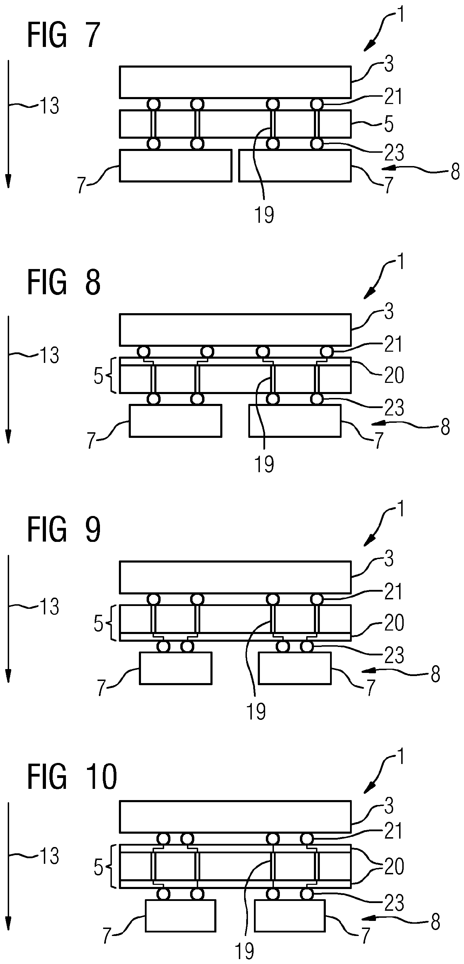

Die Fig. 7 zeigt eine beispielhafte Ausführung des erfindungsgemäßen Röntgendetektors 1 gemäß einer fünften Ausführungsform in einer Seitenansicht. Die Zwischenschicht 5 weist eine Durchkontaktierung 19 der elektrisch leitenden Verbindungen von der dem Konverterelement 3 zugewandten Seite der Zwischeneinheit 5 zur der Auswerteebene 8 zugewandten Seite der Zwischeneinheit 5 auf.

Die Fig. 8 zeigt eine beispielhafte Ausführung des erfindungsgemäßen Röntgendetektors 1 gemäß einer sechsten Ausführungsform in einer Seitenansicht. Die Zwischenschicht 5 weist eine Umverdrahtung 20 an der dem Konverterelement 3 zugewandten Seite der Zwischeneinheit 5 auf. Die Zwischeneinheit 5 ist mehrlagig ausgestaltet. Die Auswerteeinheiten 7 weisen jeweils eine kleinere flächige Erstreckung als in der fünften Ausführungsform auf. Durch die Umverdrahtung 20 ist die flächige Verteilung der elektrisch leitenden Verbindungen 23 gegenüber der flächigen Verteilung der elektrisch leitenden Verbindungen 21 verändert, beispielsweise reduziert.

Die Fig. 9 zeigt eine beispielhafte Ausführung des erfindungsgemäßen Röntgendetektors 1 gemäß einer siebten Ausführungsform in einer Seitenansicht. Die Zwischenschicht 5 weist eine Umverdrahtung 20 an der Auswerteebene 8 zugewandten Seite der Zwischeneinheit 5 auf.

Die Fig. 10 zeigt eine beispielhafte Ausführung des erfindungsgemäßen Röntgendetektors 1 gemäß einer achten Ausführungsform in einer Seitenansicht. Die Zwischenschicht 5 weist eine Umverdrahtung 20 an der dem Konverterelement 3 zugewandten Seite der Zwischeneinheit 5 und an der Auswerteebene 8 zugewandten Seite der Zwischeneinheit 5 auf.

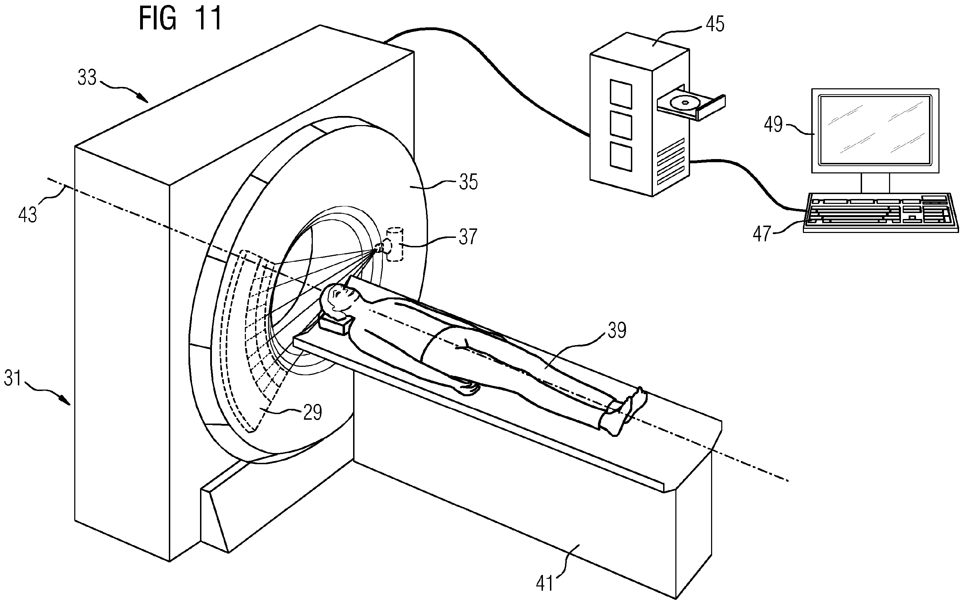

Die Fig. 11 zeigt eine beispielhafte Ausführungsform des erfindungsgemäßen Computertomographen 31 mit einer Detektorvorrichtung 29. Die Detektorvorrichtung 29 weist den erfindungsgemäßen Röntgendetektor auf. Der Computertomograph 31 beinhaltet eine Gantry 33 mit einem Rotor 35. Der Rotor 35 umfasst eine Röntgenquelle 37 und die erfindungsgemäße Detektorvorrichtung 29. Der Patient 39 ist auf der Patientenliege 41 gelagert und ist entlang der Rotationsachse z 43 durch die Gantry 33 bewegbar. Zur Steuerung und Berechnung der Schnittbilder wird eine Recheneinheit 45 verwendet. Eine Eingabeeinrichtung 47 und eine Ausgabevorrichtung 49 sind mit der Recheneinheit 45 verbunden.

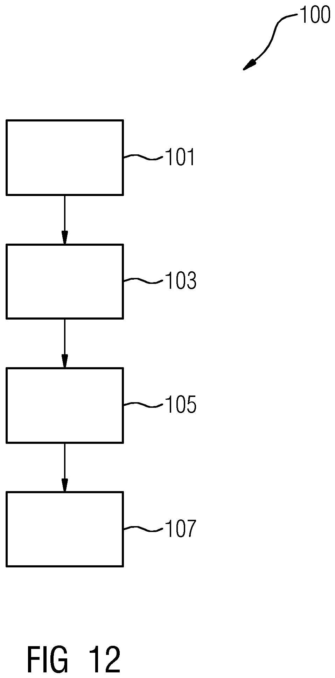

Die Fig. 12 zeigt eine beispielhafte Ausführung des erfindungsgemäßen Verfahrens 50 zur Herstellung eines erfindungsgemäßen Röntgendetektors. Das Verfahren 100 zur Herstellung eines erfindungsgemäßen Röntgendetektors weist die Schritte des Anordnens 101 und des Verbindens 103 auf. Das Verfahren 100 weist ferner den Schritt des Füllens 105 auf. Das Verfahren 100 kann ferner den Schritt des Verfestigens 107 aufweisen. Im Schritt des Anordnens 101 werden das Konverterelement, der Zwischeneinheit und die dem Konverterelement zugeordneten Mehrzahl von Auswerteeinheiten in einer Stapelanordnung angeordnet, wobei die Mehrzahl von Auswerteeinheiten in einer Auswerteebene angeordnet ist. Im Schritt des elektrisch leitenden Verbindens 103 werden das Konverterelement und die Mehrzahl von Auswerteeinheiten elektrisch leitend verbunden. Im Schritt des Füllens 105 werden die Zwischenräumen zwischen dem Konverterelement, dem Zwischenelement und der Mehrzahl von Auswerteeinheiten mit einem Füllmaterial gefüllt. Im Schritt des Verfestigens 107 wird das Füllmaterial verfestigt.

Im Schritt des Anordnens 101 werden das Konverterelement, die Zwischeneinheit und die Mehrzahl von Auswerteeinheiten und gegebenenfalls die etwaige Trägereinheit zueinander in einem Stapelaufbau angeordnet. Im Schritt des elektrisch leitenden Verbindens 103 werden das Konverterelement, die Zwischeneinheit und die Mehrzahl von Auswerteeinheiten, beispielsweise mittels Lotverbindungen oder elektrisch leitender Klebeverbindung, verbunden. Der Schritt des elektrisch leitenden Verbindens 103 kann ferner das elektrisch leitende Verbinden der Mehrzahl von Auswerteeinheiten mit der etwaigen Trägereinheit umfassen. Es sind Zwischenräume zwischen dem Konverterelement und der Zwischeneinheit bzw. der Zwischeneinheit und der Auswerteeinheit bzw. der Auswerteeinheit und der etwaigen Trägereinheit ausgebildet, welche im etwaigen Schritt des Füllens 105 gefüllt werden. Es kann ferner an der dem Konverterelement abgewandten Seite der Auswerteeinheit ein Substrat oder eine Trägereinheit angeordnet sein, welches beispielsweise mittels Lotverbindungen oder einer elektrisch leitenden Klebeverbindung mit der Mehrzahl von Auswerteeinheiten mittel des elektrisch leitenden Verbindens 103 verbunden ist. Zwischen der Auswerteeinheit und dem Substrat kann ein Zwischenraum ausgebildet sein, welcher im etwaigen Schritt des Füllens 105 gefüllt werden kann.

Das Anordnen 101 und das elektrisch leitende Verbinden 103 können schrittweise erfolgen. Die Schritte des Anordnens 101 und des elektrisch leitenden Verbindens 103 können dabei mehrmals aufeinander folgen, so dass der Stapelaufbau ebenenweise bzw. schichtweise ausgebildet wird.

Der Schritt des elektrisch leitenden Verbindens 103 kann insbesondere ein Stufenlötverfahren umfassen. Das elektrisch leitende Verbinden 103 kann schrittweise erfolgen, so dass der Stapelaufbau in mehreren Verbindungsschritten ausgebildet wird. Dabei können unterschiedliche Verbindungstechniken, beispielsweise Lotverbindung oder Klebeverbindung, verwendet werden. Die elektrisch leitenden Verbindungen können entlang der Stapelrichtung unterschiedliche Materialzusammensetzungen und Eigenschaften, beispielsweise eine unterschiedliche Schmelztemperatur, aufweisen. Es kann ein sogenanntes Stufenlöten zum elektrisch leitenden Verbinden genutzt werden, wobei die Ebenen des Stapelaufbaus nacheinander elektrisch leitend mittels Lotverbindungen mit unterschiedlicher Schmelztemperatur verbunden werden. Bevorzugt kann die niedrigste Schmelztemperatur zwischen dem Konverterelement und der Zwischeneinheit verwendet werden. Beispielsweise kann ein sogenannter Reflow-Lötprozess verwendet werden.

Im Schritt des Füllens 105 werden die Zwischenräumen zwischen dem Konverterelement, dem Zwischenelement und der Mehrzahl von Auswerteeinheiten, und gegebenenfalls zwischen der Mehrzahl von Auswerteeinheiten und der etwaigen Trägereinheit, mit einem Füllmaterial gefüllt. Im Schritt des Füllens 105 kann das Füllmaterial in einem fließfähigen Zustand in den Zwischenraum bzw. die Zwischenräume eingefüllt werden. Das Füllen 105 kann gleichzeitig für alle Zwischenräume einer Ebene bzw. Schicht der Unterfüllung oder schrittweise erfolgen.

Das Füllen 105 kann zwischen den verschiedenen Ebenen des Stapelaufbaus, also zwischen dem Konverterelement, dem Zwischenelement und der Mehrzahl von Auswerteeinheiten, und gegebenenfalls zwischen der Mehrzahl von Auswerteeinheiten und der etwaigen Trägereinheit, gleichzeitig oder schrittweise, insbesondere nacheinander, eingefüllt werden. Das Füllmaterial ist für die Zwischenräume unterschiedlicher Ebenen unterschiedlich. Beispielsweise kann in den Zwischenräumen zwischen dem Konverterelement und der Zwischeneinheit, dem Zwischenelement und der Mehrzahl von Auswerteeinheiten, und gegebenenfalls zwischen der Mehrzahl von Auswerteeinheiten und der etwaigen Trägereinheit, ein unterschiedliches Füllmaterial verwendet werden. Die unterschiedlichen Füllmaterialien können sich beispielsweise durch Viskosität im fließfähigen Zustand oder/und im verfestigten Zustand, Wärmeleitfähigkeit, thermischem Ausdehnungskoeffizient, Transparenz für Licht im sichtbaren, ultravioletten oder infraroten Bereich oder ähnlichen unterscheiden.

Das Verfestigen 107 kann gleichzeitig oder schrittweise bzw. ebenenweise erfolgen. Im Schritt des Verfestigens 107 kann das Füllmaterial beispielsweise mittel thermischer Einwirkung oder unter Einwirkung von UV-Strahlung ausgehärtet werden. Durch das Härten wird die Unterfüllung erzeugt. Die Unterfüllung weist im Endzustand ein verfestigtes Füllmaterial auf.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den durch die Ansprüche bestimmten Schutzumfang der Erfindung zu verlassen.

## Patentansprüche

1. Röntgendetektor (1) aufweisend ein Konverterelement (3), eine Zwischeneinheit (5, 5') und eine dem Konverterelement (3) zugeordnete Mehrzahl von Auswerteeinheiten (7), wobei
a. die Mehrzahl von Auswerteeinheiten (7) in einer Auswerteebene (8) angeordnet ist,
b. das Konverterelement (3), die Zwischeneinheit (5, 5') und die Auswerteebene (8) in einer Stapelanordnung angeordnet sind,
c. das Konverterelement (3) und die Mehrzahl von Auswerteeinheiten (7) mittels elektrisch leitender Verbindungen (21, 23) elektrisch leitend verbunden sind,
d. die Zwischenräume (16) zwischen dem mindestens einen Konverterelement (3), der Zwischeneinheit (5, 5') und der Mehrzahl von Auswerteeinheiten (7) mit einem Füllmaterial (18) ausgefüllt sind, wobei die Unterfüllung (17) zwischen dem Konverterelement (3) und der Zwischeneinheit (5, 5') und die Unterfüllung (17) zwischen der Zwischeneinheit (5, 5') und der Mehrzahl an Auswerteeinheiten (7) getrennt ausgebildet ist, **dadurch gekennzeichnet dass**
das Füllmaterial (18) für die Zwischenräume (16) unterschiedlicher Ebenen unterschiedlich ist.

2. Röntgendetektor (1) nach einem der vorangehenden Ansprüche, wobei der Stapelaufbau ferner eine Trägereinheit (9) umfasst.

3. Röntgendetektor (1) nach einem der vorangehenden Ansprüche, wobei die Zwischeneinheit (5, 5') und das mindestens eine Konverterelement (3) eine im Wesentlichen gleiche flächige Erstreckung (50, 50') aufweisen.

4. Röntgendetektor (1) nach einem der vorangehenden Ansprüche, wobei die Zwischenschicht (5, 5') eine Durchkontaktierung (19) der elektrisch leitenden Verbindungen (21, 23, 25) von der dem Konverterelement (3) zugewandten Seite der Zwischeneinheit (5, 5') zur der Auswerteebene (8) zugewandten Seite der Zwischeneinheit (5, 5') aufweist.

5. Röntgendetektor (1) nach einem der vorangehenden Ansprüche, wobei die Zwischenschicht (5, 5') eine Umverdrahtung (20) an der dem Konverterelement (3) zugewandten Seite der Zwischeneinheit (5, 5') oder/und der Auswerteebene (8) zugewandten Seite der Zwischeneinheit (5, 5') aufweist.

6. Röntgendetektor (1) nach Anspruch 5, wobei die Zwischeneinheit (5, 5') mehrlagig ausgestaltet ist.

7. Medizinisches Gerät aufweisend einen Röntgendetektor (1) nach einem der vorangehenden Ansprüche.

8. Medizinisches Gerät nach Anspruch 7, wobei das medizinische Gerät ein Computertomographiesystem (31) ist.

9. Verfahren (100) zur Herstellung eines Röntgendetektors (1) nach einem der Ansprüche 1 bis 6 aufweisend die Schritte:
a. Anordnen (101) von dem Konverterelement (3), der Zwischeneinheit (5, 5') und der dem Konverterelement (3) zugeordneten Mehrzahl von Auswerteeinheiten (7) in einer Stapelanordnung, wobei die Mehrzahl von Auswerteeinheiten (7) in einer Auswerteebene (8) angeordnet ist,
b. elektrisch leitendes Verbinden (103) des Konverterelements (3) und der Mehrzahl von Auswerteeinheiten (7), und
c. Füllen (105) von Zwischenräumen (16) zwischen dem Konverterelement (3), dem Zwischenelement (5, 5') und der Mehrzahl von Auswerteeinheiten (7) mit einem Füllmaterial (18) wobei die Unterfüllung (17) zwischen dem Konverterelement (3) und der Zwischeneinheit (5, 5') **dadurch gekennzeichnet dass**
die Unterfüllung (17) zwischen der Zwischeneinheit (5, 5') und der Mehrzahl an Auswerteeinheiten (7) getrennt ausgebildet wird.

10. Verfahren nach Anspruch 9, wobei das Anordnen (101) und das elektrisch leitende Verbinden (103) schrittweise erfolgen.

11. Verfahren (100) nach Anspruch 10, wobei der Schritt das elektrisch leitende Verbinden (103) ein Stufenlötverfahren umfasst.

12. Verfahren (100) nach einem der Ansprüche 9 bis 11, wobei das Füllen (105) schrittweise erfolgt.

13. Verfahren (100) nach einem der Ansprüche 9 bis 12, ferner aufweisend den Schritt:
d. Verfestigen (107) des Füllmaterials (18).

## Claims

1. X-ray detector (1) having a converter element (3), an intermediate unit (5, 5') and a plurality of evaluation units (7) assigned to the converter element (3), wherein
a. the plurality of evaluation units (7) is arranged in an evaluation level (8),
b. the converter element (3), the intermediate unit (5, 5') and the evaluation level (8) are arranged in a stack arrangement,
c. the converter element (3) and the plurality of evaluation units (7) are connected in an electrically-conducting manner by means of electrically-conducting connections (21, 23),
d. the interspaces (16) between the at least one converter element (3), the intermediate unit (5, 5') and the plurality of evaluation units (7) are filled with a filler material (18), wherein the underfilling (17) between the converter element (3) and the intermediate unit (5, 5') and the underfilling (17) between the intermediate unit (5, 5') and the plurality of evaluation units (7) is embodied separately,
**characterised in that**
the filler material (18) is different for the interspaces (16) of different levels.

2. X-ray detector (1) according to one of the preceding claims, wherein the stack structure further comprises a carrier unit (9).

3. X-ray detector (1) according to one of the preceding claims, wherein the intermediate unit (5, 5') and the at least one converter element (3) have a substantially uniform planar extension (50, 50').

4. X-ray detector (1) according to one of the preceding claims, wherein the interlayer (5, 5') has through-contacting (19) of the electrically-conducting connections (21, 23, 25) from the side of the intermediate unit (5, 5') facing the converter element (3) to the side of the intermediate unit (5, 5') facing the evaluation level (8).

5. X-ray detector (1) according to one of the preceding claims, wherein the interlayer (5, 5') comprises rewiring (20) on the side of the intermediate unit (5, 5') facing the converter element (3) and/or the side of the intermediate unit (5, 5') facing the evaluation level (8) .

6. X-ray detector (1) according to claim 5, wherein the intermediate unit (5, 5') is embodied in several layers.

7. Medical device having an X-ray detector (1) according to one of the preceding claims.

8. Medical device according to claim 7, wherein the medical device is a computed tomography system (31).

9. Method (100) for producing an X-ray detector (1) according to one of claims 1 to 6 having the steps:
a. arrangement (101) of the converter element (3), the intermediate unit (5, 5') and the plurality of evaluation units (7) assigned to the converter element (3) in a stack arrangement, wherein the plurality of evaluation units (7) is arranged in an evaluation level (8), and
b. connection in an electrically conducting manner (103) of the converter element (3) and the plurality of evaluation units (7), and
c. filling (105) interspaces (16) between the converter element (3), the intermediate element (5, 5') and the plurality of evaluation units (7) with a filler material (18), wherein the underfilling (17) between the converter element (3) and the intermediate unit (5, 5'), **characterised in that** the underfilling (17) between the intermediate unit (5, 5') and the plurality of evaluation units (7) is embodied separately

10. Method according to claim 9, wherein the arrangement (101) and the connection in an electrically conducting manner (103) are performed gradually.

11. Method (100) according to claim 10, wherein the step of connection in an electrically conducting manner (103) includes a step-soldering process.

12. Method (100) according to one of claims 9 to 11, wherein the filling (105) is performed gradually.

13. Method (100) according to one of claims 9 to 12, further having the step:
d. solidification (107) of the filler material (18) .

## Revendications

1. Détecteur (1) de rayons X comportant un élément (3) convertisseur, une unité (5, 5') intermédiaire et une pluralité, associée à l'élément (3) convertisseur, d'unités (7) d'exploitation, dans lequel,
a. la pluralité d'unités (7) d'exploitation est disposée dans un plan (8) d'exploitation,
b. l'élément (3) convertisseur, l'unité (5, 5') intermédiaire, et le plan (8) d'exploitation sont disposés suivant un agencement en empilement,
c. l'élément (3) convertisseur et la pluralité d'unités (7) d'exploitation sont connectés d'une manière conductrice de l'électricité au moyen de lignes (21, 23) conductrices de l'électricité,
d. les espaces (16) intermédiaires entre le moins un élément (3) convertisseur, l'unité (5, 5') intermédiaire et la pluralité d'unités (7) d'exploitation sont remplis d'un matériau (18) de charge, le sous remplissage (17) entre l'élément convertisseur et l'unité (5, 5') intermédiaire et le sous remplissage (17) entre l'unité (5, 5') intermédiaire et la pluralité d'unité (7) d'exploitation étant constitués de manière distincte.
**caractérisé en ce que**
le matériau (18) de remplissage des espaces (16) intermédiaires de plans différents est différent.

2. Détecteur (1) de rayons X suivant l'une des revendications précédentes, dans lequel la structure en pile comprend en outre une unité (9) de support.

3. Détecteur (1) de rayons X suivant l'une des revendications précédentes, dans lequel l'unité (5, 5') intermédiaire et le au moins un élément (3) convertisseur ont une étendue (50, 50') en surface sensiblement égale.

4. Détecteur (1) de rayons X suivant l'une des revendications précédentes, dans lequel la couche (5, 5') intermédiaire a un contact (19) traversant des connexions (21, 23, 25) conductrices de l'électricité, du côté, tourné vers l'élément (3) convertisseur, de l'unité (5, 5') intermédiaire au côté, tourné vers le plan (8) d'exploitation, de l'unité (5, 5') intermédiaire.

5. Détecteur (1) de rayons X suivant l'une des revendications précédentes, dans lequel la couche (5, 5') intermédiaire a un recâblage (20) du côté, tourné vers l'élément (3) convertisseur, de l'unité (5, 5') intermédiaire ou/et du côté, tourné vers le plan (8) d'exploitation, de l'unité (5, 5') intermédiaire.

6. Détecteur (1) de rayons X suivant la revendication 5, dans lequel l'unité (5, 5') intermédiaire est constituée de plusieurs couches.

7. Appareil médical ayant un détecteur (1) de rayons X suivant l'une des revendications précédentes.

8. Appareil médical suivant la revendication 7, dans lequel l'appareil médical est un système (31) de tomodensitométrie assistée par ordinateur.

9. Procédé (100) de fabrication d'un détecteur (1) de rayons X suivant l'une des revendications 1 à 6, comportant les stades :
a. on met (101) l'élément (3) convertisseur, l'unité (5, 5') intermédiaire et la pluralité associée à l'élément (3) convertisseur, d'unités (7) d'exploitation en un agencement en empilement, la pluralité d'unités (7) d'exploitation étant mise dans un plan (8) d'exploitation,
b. on connecte (103) d'une manière conductrice de l'électricité, l'élément (3) convertisseur et la pluralité d'unités (7) d'exploitation,
c. on remplit (105) des espaces (16) intermédiaires entre l'élément (3) convertisseur, l'élément (5, 5') intermédiaire et la pluralité d'unités (7) d'exploitation d'un matériau (8) de charge, le sous remplissage (17) entre l'élément (3) convertisseur et l'unité (5, 5') intermédiaire étant **caractérisée en ce qu'**il est séparé du sous remplissage (17) entre l'unité (5, 5') intermédiaire et la pluralité d'unités (7) d'exploitation.

10. Procédé suivant la revendication 9, dans lequel la mise (101) et la connexion (103) conductrice de l'électricité s'effectue pas à pas.

11. Procédé suivant la revendication 10, dans lequel le stade de la connexion (103) conductrice de l'électricité comprend un procédé de brasage par étape.

12. Procédé suivant l'une des revendications 9 à 11, dans lequel le remplissage (105) s'effectue pas à pas.

13. Procédé suivant l'une des revendications 9 à 12, comprenant en outre le stade :
d. durcissement (107) du matériau (18) de charge.
